# EUROPEAN PATENT SPECIFICATION

(11) **EP 1 937 160 B1**
(45) Date of publication and mention of the grant of the patent: **27.04.2011**
(21) Application number: 06792229.4
(22) Date of filing: 22.09.2006
(51) Int. Cl.: A61B 17/16, A61B 17/17, A61B 17/00

(54) **A SURGICAL DRILL, A SET OF SURGICAL DRILLS AND A SYSTEM FOR CUTTING BONE**
CHIRURGISCHER BOHRER, SET VON CHIRURGISCHEN BOHRERN UND SYSTEM ZUM SCHNEIDEN VON KNOCHEN
FORET CHIRURGICAL, ENSEMBLE DE FORETS CHIRURGICAUX ET SYSTEME POUR SECTIONNER UN OS

(30) Priority: 23.09.2005 GB 0519459
(43) Date of publication of application: 02.07.2008
(73) Proprietor: Hoogland, Thomas, 85774 Unterföhring (DE)
(72) Inventor: Hoogland, Thomas, 85774 Unterföhring (DE)
(74) Representative: HOFFMANN EITLE
(86) International application number: PCT/EP2006/009227
(87) International publication number: WO 2007/039141

(56) References cited:
- EP-A- 1 402 843
- WO-A-94/27507
- FR-A- 2 828 397
- US-A- 5 342 366
- US-A- 5 505 732
- US-A- 5 573 537
- US-A- 5 931 841
- US-A1- 2004 131 993
- US-B1- 6 224 607
- US-B1- 6 689 125

## Description

### Field of the Invention

The present invention relates to a surgical drill according to the pre-characterizing part of claim 1, a set of surgical drills, and a system for cutting bone.

In the field of spinal surgery is a need to expose the intraspinal structures by removing parts of the facet joints and the facet joint capsule by a posterior or posterolateral approach. This removal of parts of the bone structure of the spine is carried out in order to access the spinal canal which is filled with nerve structures. The nerve structures in the spinal canal have a weak tissue structure and a tree-like configuration such that the central part of the spinal canal is filled with a long sack (dura) filled with nerve fibres. From this long sack filled with nerves, nerve roots are branching off at intervertebral levels to the left and to the right, whereas the nerve roots are leaving the spinal canal through bilateral tunnels, the foramen.

In the human being, the dural sack comprising the nerve fibres and its branches can be pathologically compressed at the level of the spinal canal or inside the foramen (tunnels) by:
1. protruded or extruded disc material
2. hypertrophied facet joints
3. hypertrophied facet joint capsule
4. synovial cysts from the facet joints
5. intraspinal tumors or
6. disc collapses that may cause narrowing of the foramen.

Such compression of the dural sack and the respective nerve fibres inside the dural sack causes pain, numbness or even paralysis of the patient. The most common complication is that of a herniated vertebral disc.

To access the spinal canal in order to treat or remove the pathological tissue, the surrounding bone material has to be removed. By removing parts of the facet joint capsule and parts of the facet joint bone an opening of 6 to 11 mm diameter can be created through which a working cannula can be inserted. Through this working cannula the surgeon can manipulate the pathological interspinal tissue and can, for example, remove such a tissue with standard instruments like graspers, curettes, reamers, lasers etc. Through such a working cannula, also a cleaning of the interdiscal space can be performed and, if necessary, interdiscal spacers or cages can be inserted. After a working cannula is introduced, the removal of pathological structures in the spinal canal can, furthermore, be performed with aid of a endoscope.

Such surgery is usually carried out under the control of an two-direction X-ray image intensifier.

The removal of the bone structure to access the spinal canal is a very delicate and sensitive procedure. It has to be avoided by any means that the nerve sack (dura) or one of the branching nerve roots is injured or severed since such an injury is very dangerous and the patient may not recover.

A standard procedure for opening the spinal canal has been using surgical drills or milling cutters to introduce an opening in the facet joint capsule and/or the facet joints to open up the spinal canal. These drills have to be advanced extremely carefully in order not to injure, or even sever, the intraspinal tissue structures.

A a vertebral drill bit and inserter is disclosed in US 6,443,956.

US 5,931,841 pertains to a combination broacher-reamer for use in orthopaedic surgery, in particular for the installation of prosthetic hips and knees, requiring that the intramedullary canal of the femur be hollowed in order to accept the stem of a prosthetic implant.

US 5,573,537 relates to an instrument for probing and reaming a pedicle for use in spinal fixation surgery.

WO 94/27507 A1 relates to a flexible medullary reaming system for preparing a long bone, such as a femur, to receive a prosthesis, such as a total hip replacement.

### Summary

Thus, it is an objective of the present invention to provide a surgical drill and a system for removing bone which increases the reliability in terms of the protection of the intraspinal tissues.

This objective is solved by a surgical drill according to claim 1. Due to the provision of said non-cutting protection tip, nerves, tissue and dura are protected during driving the drill. Since the protection tip is constructed and arranged to be non-cutting, any contact with nerve structures, tissue or dura inside the spinal canal leads to a displacement of the respective soft tissue only. Due to said non-cutting protective tip, a severing or injuring of the delicate soft tissue inside the spinal canal can be reliably excluded.

The terms "distal" and "proximal" are used in this application in the sense that is usually assigned to them in connection with surgical instruments, namely the term "distal" indicating the end of a surgical instrument remote from the surgeon when the instrument is in use and the term "proximal" indicating the end of the surgical instrument that is near to the surgeon when the surgical instrument is in use. In other words, the distal end is the end of the surgical instrument which is inserted first into a patient's body and which typically is the end for manipulating the respective surgery site and the proximal end being in the hands of the surgeon.

In one preferred embodiment of the surgical drill, the cutting section has a first outer diameter and the non-cutting protection tip has a second outer diameter which is smaller than the first outer diameter of the cutting section. In other words, the diameter of the non-cutting protection tip is smaller than the diameter of the cutting section. In a preferred embodiment the difference in diameter is 1 mm to 2 mm. An advantage of this preferred embodiment is that the non-cutting protection tip can be inserted into a pilot channel or other channel that has been established by a previous drilling device before using the actual surgical drill. Accordingly, the non-cutting protection tip does not only serve the purpose of protecting the delicate soft tissue inside the spinal canal but also serves as a guide means for guiding the surgical drill in the direction of a pre-cut pilot channel through the bone. This has the advantage that the cutting procedure with the present surgical drill is easy to carry out since the surgeon can completely focus on the pressure and drilling speed executed on the drill and does not have to concentrate on centring, angling and aiming the drill. Furthermore, as will be explained below with regard to the set of surgical drills and the system of surgical drills, with such a drill it is convenient to extend an already existing drilled channel successively such that a desired diameter is reached after several drilling steps.

The non-cutting protection tip of the surgical drill comprises in one preferred embodiment at least one substantially cylindrical section and at least one substantially tapered section situated distally of the cylindrical section. In a further preferred embodiment, the non-cutting protection tip comprises at least one substantially rounded section situated distally of the substantially tapered section. The provision of the cylindrical section enables a good centring about the axis of the surgical drill whereas the tapered section helps to insert the non-cutting protection tip into a readily drilled channel in the bone structure. This embodiment enables the surgeon to insert and center the surgical drill reliably with regard to an already present channel. The rounded section of the non-cutting protection tip ensures that the nerves, tissue, and/or dura are protected during the cutting process.

The non-cutting protection tip preferably is blunt. Preferably, the non-cutting protection tip has at least one polished and/or smooth surface section, in particular a mechanically polished surface section and/or an electro-polished surface section. In a preferred embodiment the non-cutting protection tip and/or the drill member has a smooth finishing and/or coating, in particular a lubricious coating and/or a low friction coating, in particular an PTFE coating. The provision of a blunt, smooth surface, which is possibly coated with a non-sticking material, leads to an even better protection of the delicate tissue inside the spinal canal since such a tissue will not be injured or stretched by sticking to the tip of the drill.

In order to provide the advantages of the non-cutting protection tip, it is preferred that the protection tip has a length in the distal direction (axial direction of the drilling means) of at least 5 mm, preferably between 5 and 15 mm and preferred of 5 mm, 10 mm or 15 mm. These dimensions provide a tip which provides reliable guidance through a previously-cut canal in the bone and, on the other hand, do not extend too far inside the spinal canal when the drilling is finished and the cutting section necessarily enters the spinal canal for a short distance. The choice of the length of the protective non-cutting tip depends on the actual anatomy of the respective patient, in particular on the size and structure of the spinal canal.

Preferred, the protective non-cutting tip is rounded and may have, for example with a cross-section of a semi-circle, an semi-ellipse or similar rounded, smooth forms.

To provide reliable cutting, the cutting section has a cutting end face adjacent to the non-cutting protection tip. The longitudinal axis of the drill member is oriented perpendicular to the cutting end face of the cutting section.

In a further preferred embodiment, the drill comprises a sleeve which has an inner diameter that is chosen such that the drill member can be easily inserted and rotated. The sleeve serves to protect the surrounding tissue when the drill is inserted into the body and protects the tissue when the drill is operated.

In a preferred embodiment, the sleeve has an inner threading on its proximal end and the drill member has an outer threading on its proximal end such that the drill member can be advanced relative to the sleeve by relative rotation of the drill member. By this arrangement, the feed rate of the drilling member relative to the sleeve can be exactly controlled. This is helpful in particular when the cutting section does not have a self-cutting drill-threading but is rather of the type of a milling cutter.

The drill member of the surgical drill has a co-axial guide channel inside of the drill member which extends in the axial direction such that a guide wire can be inserted into the guide channel. The guide channel extends from the distal end of the drill member to the proximal end of the drill member. Such a guide wire, as will be explained below, is used to guide the drill member along the guide wire into the desired position inside the patients' body and is particularly helpful to increase the precision and to reduce the duration of a surgical procedure.

The cutting section of the drill member can be, for example, a burr, a trephine, a milling cutter, a reamer or a trepan. The cutting section can also comprise a drill threading.

In a preferred embodiment, the surgical drill comprises a handle, a removable handle and/or a connector to a power drill at a proximal end of the drill member. This allows the surgeon to choose the appropriate drive means for the respective situation.

The objective of the invention is also solved by a set of surgical drills according to claim 16. Such a set comprises at least a first and a second surgical drill according to the description given above. Furthermore, the outer diameter of the cutting section of the first surgical drill is equal to, or larger than the outer diameter of the non-cutting protection tip of the second surgical drill. As has been explained above, such a configuration of the two drills leads to a set of drills which can be used to successively extend an already provided pilot channel in a bone. This set of drills is used such that the first drill is introduced with its non-cutting protection tip into an already pre-drilled pilot channel in the bone. The first drill is then driven and a channel is produced having an inner diameter which corresponds to the outer diameter of the drill section of the first surgical drill. Said channel produced by the first surgical drill is centred about the pilot channel. After the first surgical drill has completed the drilling action and drilled a channel with a diameter corresponding to the outer diameter of the cutting section of the first drill, the second drill is inserted, with its non-cutting protective tip, into the channel produced by the first drill. Then, this second drill is driven in order to expand the channel drilled by the first drill. The channel resulting from this second surgical drill has then an inner diameter which corresponds to the outer diameter of the drilling section of the second surgical drill. The channel introduced into the bone by the second surgical drill is centred about the channel that was drilled by the first surgical drill. This principle can be furthered with the required amount of different drills terminating with a surgical drill that has an outer diameter of the cutting section which corresponds to the desired inner diameter of the channel in the bone that is required to perform the actual surgery inside the bone, i.e. in the spinal canal.

In a preferred embodiment, the set of surgical drills comprises a plurality of surgical drills having cutting diameters of the cutting section of 3mm, 4mm, 5mm, 6mm, 7mm, 8mm, 9mm, 10mm, 11mm and 12mm, respectively. The diameter could also preferably be 3,5mm, 4,5mm, 5,5mm, 6,5mm, 7,5mm, 8,5mm, 9,5mm, 10,5mm, 11,5mm and 12,5mm. In a further preferred embodiment, the drills of the set of surgical drills have diameters of the cutting sections of 4mm, 6mm, 8mm and 10mm, and the diameters of the respective non-cutting protection tips of said set of surgical drills being 2mm, 4mm, 6mm and 8mm, respectively. The diameters of the non-cutting protection tip of the surgical drills have diameters such that they can be inserted into a hole drilled by the respective smaller drill of the set of surgical drills.

In a preferred embodiment, the axial lengths of the non-cutting protection tips of all surgical drills are equal.

The objective is, furthermore, solved by a system for cutting bone according to claim 22. Additionally to a surgical drill, a working cannula for insertion into the body is part of the system. The working cannula is for the insertion of surgical instruments after a channel of an appropriate diameter has been created.

In a preferred embodiment, the system furthermore comprises a needle, in particular an 18 gage needle, for positioning a first channel through the tissue of a body and, furthermore, comprises a guide wire to be inserted into this channel. The use of such a needle makes it easier to provide a channel through the tissue that surrounds the bone and a guiding wire in order to correctly align the direction for introducing a pilot channel or a first drilling channel.

The system comprises, in another preferred embodiment, a dilating rod for the dilation of a channel in a body. Due to this additional dilating rod, the channel introduced into the body can be dilated such that finally the sleeve of the first surgical drill can be introduced.

According to a method for using the surgical drill, the removal of bone includes the steps of introducing a pilot channel of a starting diameter into the bone, advancing a surgical drill having a cutting diameter larger than the starting diameter through the pilot channel, the surgical drill having a non-cutting protective tip at its distal end of a diameter equal or smaller than that of the starting diameter of the pilot channel and said non-cutting protective tip being inserted into the pilot channel before drilling. The method further comprising advancing a sequence of surgical drills of increasing cutting diameters through the channel created by the respective foregoing surgical drill whereby the diameters of the respective non-cutting protective tips are equal to or smaller than the cutting diameters of the respective foregoing surgical drill whereas the non-cutting protective tips are inserted into the channel created by the cutting section of the respective foregoing surgical drill.

In a preferred variant of the method, the pilot channel is introduced through a safe zone of the respective bone, in particular tangential to a bone canal such as the spinal canal.

In a variant of the method the pilot channel is provided by introducing a drill through the joint capsule or hypertrophied bone into a safe zone in the spinal canal lateral to the nerve sack (dura) and medial to the branching, exciting nerve root.

A third variant of the method is provided in that the drill advances into the pathological tissue such as herniated disc material, hypertrophied capsule of the facet joint or bone, synovial cyst or tumor.

In a further variant, surgical drills are advanced through the pilot channel, the surgical drills having a non-cutting protective tip in a distance of 5 mm to 15 mm from the distal-most end of the tip, whereas the cutting section has an outer diameter that is 1 mm to 2 mm larger than the diameter of the non-cutting protective tip.

In a variant of the method, a guide wire is introduced into the pilot channel and subsequently used instruments are guided by the guide wire. Using this variant, it is particularly advantageous inserting said guide wire into a guide channel that extends in the axial direction through the surgical drill.

A variant of the method proposes that before the pilot channel is introduced into the bone, a needle for opening a channel, preferably an 18 gage needle, is advanced through the tissue surrounding the bone.

In a variant a guide wire is introduced into the channel opened by the needle. Furthermore, a dilation rod can be introduced through the channel opened by the needle to dilate the channel. Advantageous, a sleeve is introduced into the dilated channel to provide a working channel through which the first surgical drill can be advanced.

### Brief Description of the Drawings

- Figure 1: is a top view of a sleeve and a surgical drill not falling within the scope of the claims;
- Figure 2: is a top view of an 18 gage needle, a dilating rod, a sleeve of a Yamshidi needle and a drill not falling within the scope of the claims;
- Figure 3: is a schematic cross-section through a surgical site in the form of an intervertebral cross- section;
- Figure 4: is a schematic cross-section through the surgical site of Figure 3 with a sleeve and a surgical drill not falling within the scope of the claims inserted into the bone structure;
- Figure 5: is a schematic cross-section of the surgical site according to Figures 3 and 4 with a drill guided by a guide wire;
- Figures 6a and b: are schematic cross-sections through a tip which is drilled into a bone;
- Figure 7: is a schematic cross-section of the surgical site as shown in Figures 3 to 5 with a working cannula introduced through the tissue;
- Figure 8a and b: are two views on two variants of the tip section of the drilling member the variant of figure 8b;
- Figure 9: is a schematic view of the proximal end section of a sleeve and a surgical drill having endwise inter engageable screw threaded portions;
- Figures 10a and 10b: are schematic side views of a surgical drill in a further embodiment;
- Figures 11a and 11b: are schematic side views of a surgical drill in a further embodiment related to that one shown in Figures 10a and 10b;
- Figure 12: is a schematic top view of the handle of the surgical drills shown in Figures 10a, 10b, 11a and 11b.

### Description of the Preferred Embodiments

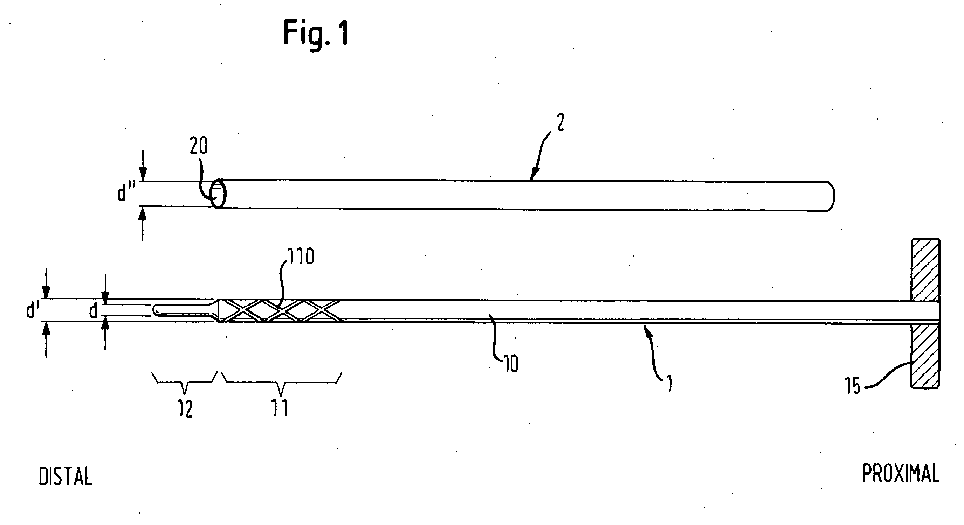

Figure 1 shows a surgical drill 1 having a drill member 10 with a cutting section 11 and a non-cutting protection tip 12. The cutting section 11 has an outer diameter d' that corresponds to the diameter that is actually cut into the bone. The cutting section 11 has, furthermore, a drill surface structure 110 for cutting the bone. The cutting section 11 is provided on a distal end of the drill member 10. The non-cutting protection tip 12 has a diameter d. The diameter d of the non-cutting protection tip 12 is smaller than the diameter d' of the cutting section 11. In particular, the difference between the diameter of the cutting section 11 and the non-cutting protection tip is d'-d = 1 mm in the embodiment shown in the Figure. All Figures are not drawn to scale.

The non-cutting protection tip 12 has a smooth, blunt surface which has a surface finish that does not stick to the intraspinal tissue. The surface finish of the non-cutting protection tip 12 may comprise a low-friction coating such as a PTFE coating and/or a lubricious coating and/or has a surface finish with similar properties.

The drill member 10 is provided with an handle 15 on its proximal end.

A sleeve 2 to cover the drill member 10 when it is inserted into the body is provided. The sleeve 2 has a sleeve channel 20 which has an inner diameter d''. The inner diameter d'' of the sleeve channel 20 is formed such that the drill member 10 of the surgical drill 1 can be inserted in and advanced through the sleeve channel 20 of the sleeve.

In a preferred embodiment of the invention, the sleeve 2 has dimensions that enable the drill member 10 to be rotated within the sleeve 2 without taking along the sleeve 2 but, on the other hand, it is preferred that the sleeve 2 has an outer diameter that is, under the aforementioned condition, as small as possible. In other words, the wall-thickness of the sleeve is preferably small. Such sleeve preferably is made of a material that allows for a low wall-thickness of the sleeve, for example stainless steel or other materials with comparable properties.

In another preferred embodiment of the invention, the sleeve 2 has an outer diameter that corresponds to the inner diameter of a subsequently inserted drill member such that the outer diameter of the sleeve 2 already dilates the tissue surrounding the sleeve in order to facilitate the insertion of a subsequent sleeve and/or drill into the body.

The sleeve 2 may have an outer surface finish that simplifies the insertion of the sleeve 2 into body tissue. Such surface finish could comprise a lubricious coating and/or a low-friction coating such as a PTFE coating.

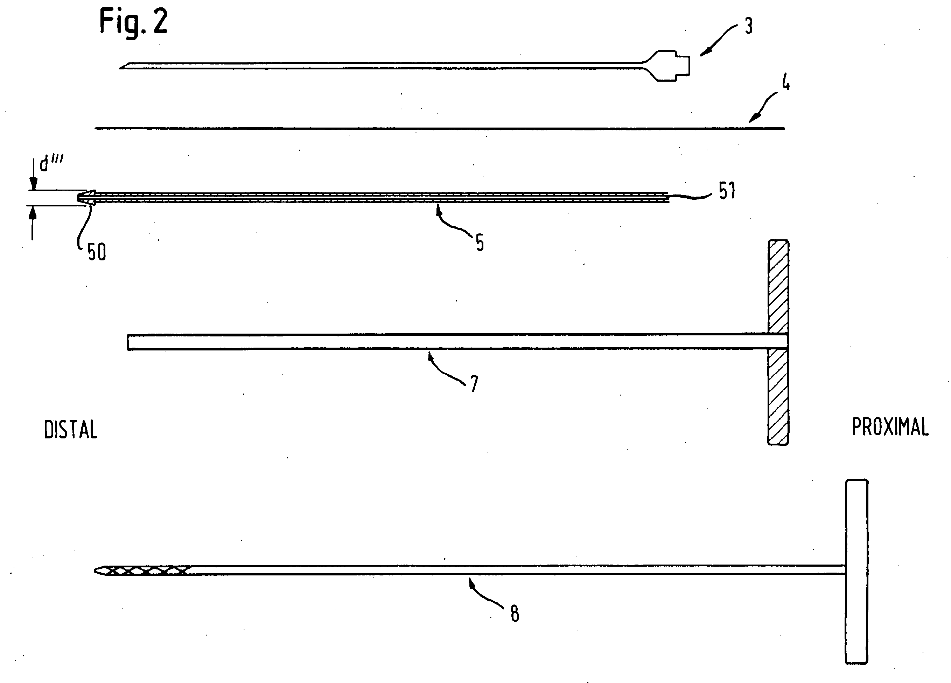

In Figure 2, further components of a system with which the surgical drill 1 and the sleeve 2 shown in Figure 1 can be used.

An 18-gage hollow needle 3 is shown in Figure 2 that is used to open up a first channel to the bone structure that is going to be worked on with the surgical drill 1 shown in Figure 1. The hollow needle 3 may include in its cavity a trocar (not shown) that can mark a pilot notch in the bone as soon as the needle 3 is inserted into the body through the tissue and is located in the right position where the drilling is intended to take place. After the pilot notch has been introduced into the bone, the trocar is retracted from the needle 3 that is still held in place and a thin guide wire 4 is inserted into the cavity of the needle 3 such that the end of the guide wire 4 touches the bone at the position of the pilot notch.

A dilating rod 5 is shown in Figure 2 which comprises a guiding channel 51 extending axially through the dilating rod 5 from the proximal end to the distal end. The guide wire 4 is inserted into the guide channel 51 of the dilating rod 5, such that the dilating rod 5 is guided along the guide wire 4 towards the bone and towards the pilot notch in the bone. The dilating rod 5 has an expanding tip 50 on its distal end that leads to a maximum diameter d''' that is preferably equal to or slightly smaller than the inner diameter of an subsequently introduced Yamshidi sleeve 7. The dilating rod dilates the tissue surrounding the bone.

Yamsihdi sleeve 7 is a part of a Yamshidi needle. Said Yamshidi sleeve 7 is put over the dilating rod 5 that serves to guide the Yamshidi sleeve 7 into the right position. Subsequently, the dilating rod is withdrawn from the Yamshidi sleeve 7 and a Yamshidi trocar (not shown) of the Yamshidi needle is inserted into the Yamshidi sleeve 7 to introduce a pilot hole into the bone structure at the position of the pilot notch and an angle determined by the surgeon. This is preferably done under a 2-dimensional x-ray. Subsequently the Yamshidi trocar is withdrawn from the Yamshidi sleeve 7 and conventional drill 8 is inserted into the Yamshidi sleeve 7 to drill a pilot channel into the bone in the position and direction of the pilot hole. After the pilot channel is drilled, the conventional drill is retracted from the Yamshidi sleeve 7 and the dilating rod 5 re-introduced into the Yamshidi sleeve 7 which is subsequently retracted from the body. The sleeve 2 of the surgical drill 1 shown in Figure 1 is put over the dilation rod 5 which is subsequently retracted from the sleeve 2. Then the drill member 10 of the surgical drill 1 is introduced into the sleeve 2 and its non-cutting protection tip is inserted into the pilot channel drilled into the bone and is subsequently operated to expand the pilot channel to the outer diameter of the cutting section 11 of the surgical drill 1.

Thus, Figure 2 shows several instruments that are used to prepare the surgical site to be ready for the introduction of the surgical drill 1 shown in Figure 1.

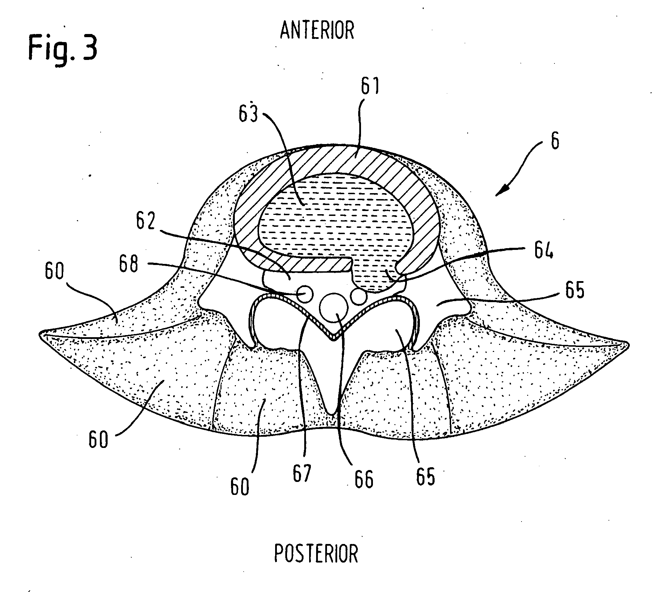

Figure 3 shows a cross-section of a spinal structure 6. Specifically, it shows the paravertebral musculature 60, the annulus of the disk 61, the spinal canal 62, the nucleus 63, a section of herniation/slipped disk 64, the facet joints 65, the dura 66 the capsule of facet joint 67 and the nerve root 68. As can be seen, the herniation 64 presses onto the dura 66 and on one of the nerve roots 68. This causes pain to the patient.

The task is, therefore, to remove this herniation and to free up the spinal canal 62 of any matter that does not belong there. In order to remove the herniation, a channel has to be opened up through the bone structure, in particular through the facet joints 65 and the capsule of the facet joint 67 into the spinal canal 62.

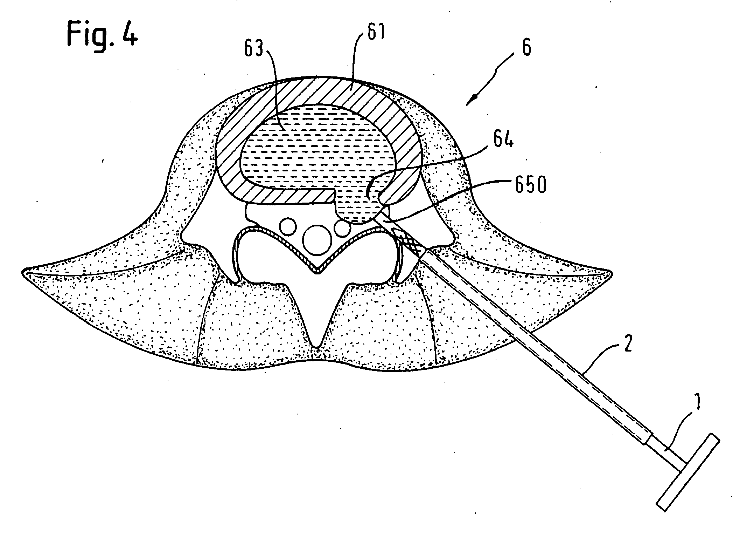

A stage of this process of opening up this channel is shown in Figure 4. In Figure 4 the spinal structure shown in Figure 3 is shown, but with a first surgical drill 1 that is surrounded by the first sleeve 2 being inserted through the paravertebral musculature 60 and readily drilled through a first part of the facet joints 65 along the pre-drilled pilot channel 650.

Then the second drilling step is prepared by retracting the first surgical drill 1 from the first sleeve, putting the second sleeve of the second surgical drill, which has a thicker diameter than that of the first sleeve, over the first sleeve and retract the first sleeve from the second sleeve. Then the second surgical drill is inserted into the second sleeve and its non-cutting protective tip is inserted into the channel drilled by the first surgical drill. The third to n-th drilling step are carried out in the same manner.

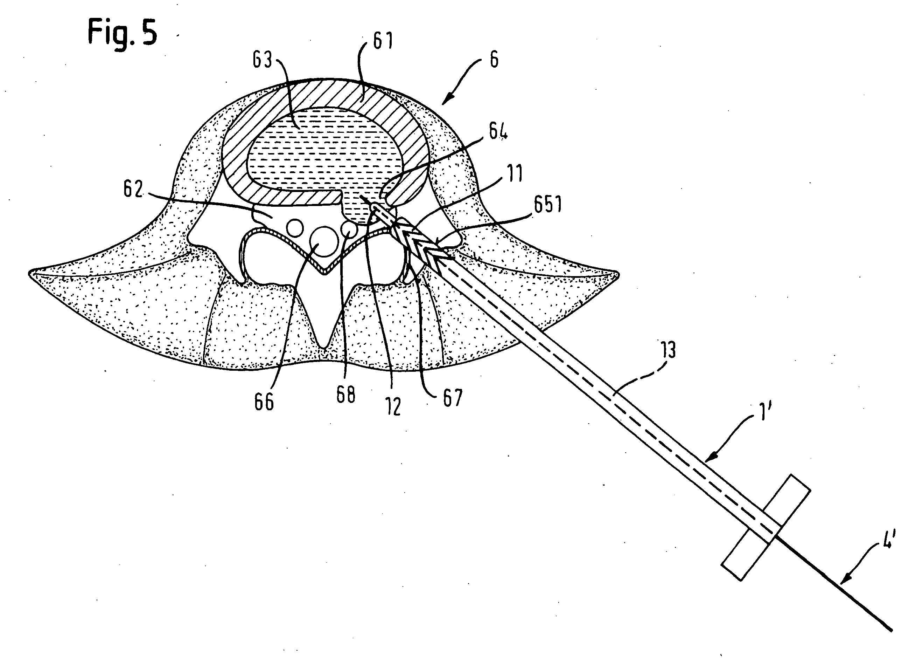

As can be seen in Figure 5, after n drilling steps, the last surgical drill 1' is inserted into the surgical site along a guide wire 4'. The guide wire 4' has been inserted into the channel 651 after the previous surgical drill was retracted from its sleeve. Then the previous sleeve is retracted as well, leaving only the - relatively thick (ca. 1-2mm)- guide wire 4' inside the body. The last surgical drill 1' has a through hole 13 that extends in axial direction from the proximal to the distal end of the drilling member 10, is put over the guide wire 4'. The last surgical drill 1' does, in a preferred embodiment, not comprise a sleeve in order to keep the outer diameter in the last drilling step as small as possible in order to keep the stress onto the surrounding tissue as low as possible.

In the stage shown in Figure 5, the surgical drill 1 has already been advanced through almost all of the facet joints 65 and the drilling section now attempts the capsule of facet joints 67. In this situation, the non-cutting protection tip 12 has already entered into the spinal canal 62 and is in contact with the herniated disk material 64. However, since the tip 12 is blunt and has a smooth surface, the herniated disk material 64 is only pushed away in this area. An actual cutting of this tissue, even though it is only pathological material, cannot happen. This increases the reliability since the pathological tissue can be removed in a controlled fashion in a subsequent step. Furthermore, in a situation where the dura 66 or the nerve roots 68 are in the way of the drilling direction, the tip 12 gently pushes this tissue away during the drilling process. In other words, since the tip 12 enters into the spinal canal first, it can keep clear all delicate tissue and nerves inside the spinal canal 66 from the sharp cutting edges of the cutting section 11.

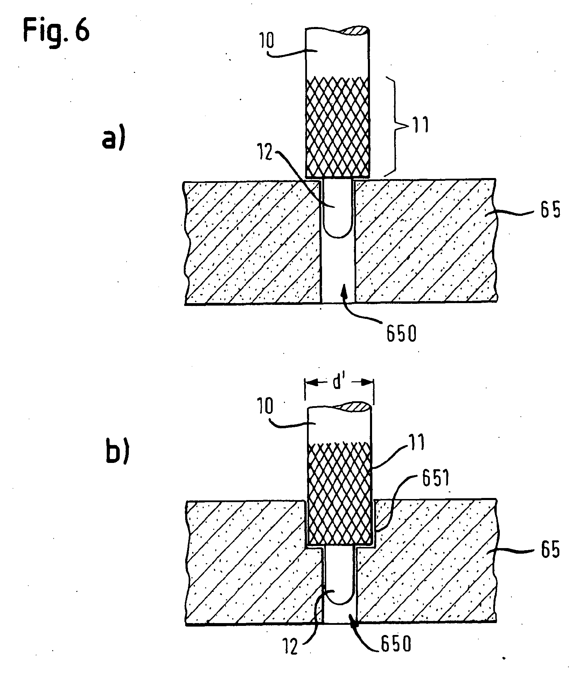

Furthermore, the non-cutting protection tip 12 helps to guide the drilling member 10 through the already existing pilot channel 650 provided within the facet joints 65. This is also shown in Figure 6. From Figure 6a) it is apparent that the non-cutting protection tip 12 is inserted into the pilot channel 650 within the facet joints 65 such that the drilling member 10 and, thus, the cutting section 11, are aligned centrally with respect to the pilot channel 650. As can be seen in Figure 6b), when advancing the drill member 10 through the facet joints 65, a expanded channel 651 results which corresponds to the outer diameter d' of the cutting section 11. The expanded channel 651 is coaxially aligned with the previously cut pilot channel 650. In this manner, the channel 651 can be opened up until the desired diameter of the channel is reached. The non-cutting protection tip 12, thereby, serves to protect the delicate tissue inside the spinal canal 62.

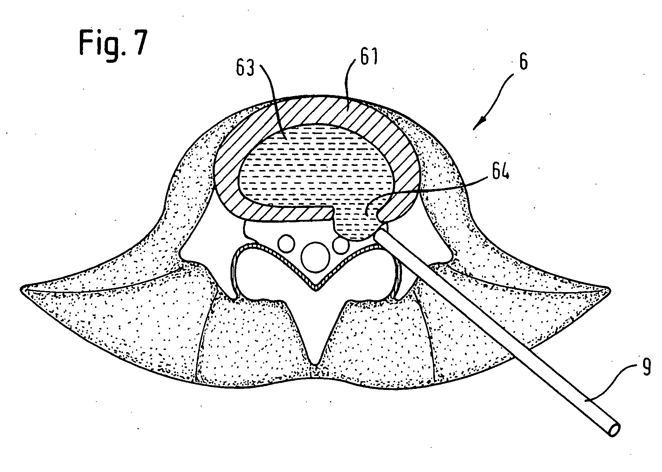

Figure 7 shows the same surgical site as Figures 3 to 5. In this Figure a working cannula 9 is introduced through the tissue into the bone such that the surgeon can insert through the working cannula 9 surgical instruments to manipulate the tissue inside of the spinal canal.

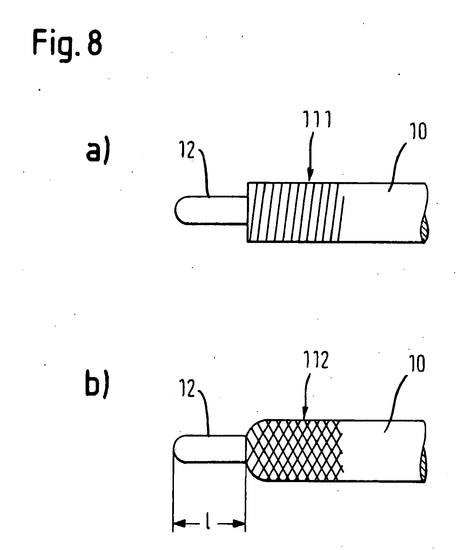

In Figure 8, two variants of tip sections of the drilling member 10 are shown. Variant a) shows a cutting section 11 that has a surface structure like a drilling threading 111. Variant b) shows a variant of the cutting section 11 that has a surface structure like a reamer 112. The tip length 1 is given between 5mm and 15 mm.

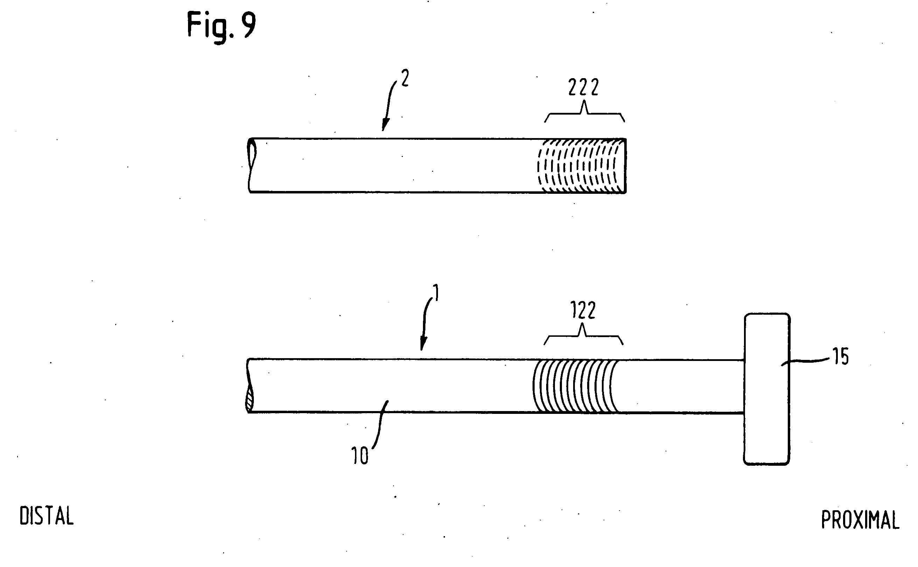

In Figure 9 the proximal end of a combination of a surgical drill 1 and a sleeve 2 is shown. The drill member 10 of the surgical drill 1 has an outer threading 122 which corresponds to an inner threading 222 of the sleeve. This configuration enables the surgeon advance the surgical drill 1 relative to the sleeve 2 in a controlled fashion. This is particularly helpful when the cutting section is of the type of a milling cutter that has no inherent threading to advance the surgical drill only by rotation.

Using the surgical instruments described above, a further method of making an exposure of various pathological tissues in the spinal canal is proposed, including the steps of:
1. introducing a first drill or needle through the joint capsule or hypertrophied bone into a safe zone in the spinal canal lateral to the nerve sack (dura) and medial to the branching, exciting nerve root, possibly into the pathological tissue being herniated disc material, hypertrophied capsule of the facet joint or bone, synovial cyst or tumor.
2. Successively advancing surgical drills 1 with a blunt tip 12 of 5 - 15 mm and a bur type cutting edge 11 as of 10 to 15 mm from the tip, having a 1 - 2 mm larger diameter at the cutting level. Surgical drills 1 that have an increasing diameter of the cutting section 11, follow the tunnel that is created by the first bur.
3. The subsequent drills are advanced through a skin opening from 6 - 11 mm penetrating bluntly the posterior lumbar muscles through a protective sleeve type cannula, which stops at the bony edges of the lumbar facet joint and lamina. Depending of the acquired size of the dorsal opening, which may vary from 6 - 12 mm a corresponding size working cannula is placed. Through this cannula a variety of spinal endoscopes and instruments can be inserted. It will be appreciated that with the method of using the drill of the present invention there is no reamer type cutting edge at the end of the tissue removing drill and that the drill has at least a 5 mm long blunt tip avoiding cutting or injuring spinal nerves or dura.

The apparatus allows safe removal of hypertrophied joint capsule and its adjacent hypertrophied ligamentum flavum (yellow ligament), as well as hypertrophied bone of the facet joint particularly in the presence of spinal stenosis. Depending on the extent of the pathology an opening up to 12 mm can be created. The procedure can be performed from one side and if desired from both sides. The latter in the presence of bilateral stenosis or in the need of insertion of a intradiscal cage. The procedure can be performed under local anaesthesia as no significant contact occurs with the spinal nerves during this procedure.

In one preferred embodiment the achievement of posterolateral opening of the spinal canal comprises of a 18 gage spinal needle that is introduced after anesthetizing the skin posterolateraly under a angle of 60 degrees and 10 - 14 cm from the midline of the dorsal spine under imaging of an X-ray intensifier directly aimed at the pathological process in the spinal canal. As soon as this spinal needle hits bony structures the trocar of the spinal needle is removed and a guide wire is inserted. Over this guide wire a dilating rod with a conical tip is introduced up to the bony structure in order to dilate the soft tissue. Subsequently a dilating cannular of 3mm is inserted and removed. Subsequently the first guiding instrument is introduced being a 3mm cannula with the 2 mm inside diameter and a holding grip.

Then, the guiding wire is removed and a standard long handbur is introduced. The first guiding instrument is used to aim the bur at the direction of the pathological structure with aid of the image intensifier in two directions. Then, the bur is advanced in the safe zone until the pathological structure is reached, creating a pilot channel of 1 - 2 cm of length and with a 2 mm diameter. Subsequently the bur is removed and replaced by guiding wire. Over the guide wire dilating cannels are advanced up to the desired diameter of the working cannula. After soft tissue dilatation up to the bone, the first surgical drill according to the invention with a 0,5 - 1,0cm long blunt tip is inserted. The surgical drill has a tip diameter of 2mm and a cutting diameter of 3mm. With the surgical drill the opening in the spinal canal is enlarged to 3mm. The special drill is then removed and a guide wire is inserted. Subsequently a blunt tip bur is introduced with a tip diameter of 3mm and a cutting diameter of 4mm. These steps are then repeated with increasing drill diameters. The drill set consists of burs with a 1 mm increase of diameters. In this fashion an opening to the spinal canal can be achieved up to 12 mm. When the desired opening c.q. tunnel diameter has been achieved a corresponding working cannula can be inserted through which the spinal endoscope and instruments can be introduced.

In order to control the surgical procedure and to be able to aim the surgical instruments correctly, several steps can be carried out under imaging of an X-ray intensifier or other suitable imaging device, namely the step of introducing the first needle that opens up the first channel through the tissue, the step of the introduction and angling of the Yamshidi needle with the step of introducing the pilot notch and the step of drilling the pilot channel to control the depth of the drilling action. After these steps have been carried out, the progress of the drilling action and the subsequent surgical work can as well frequently be controlled under the imaging of an X-ray intensifier or other suitable imaging device.

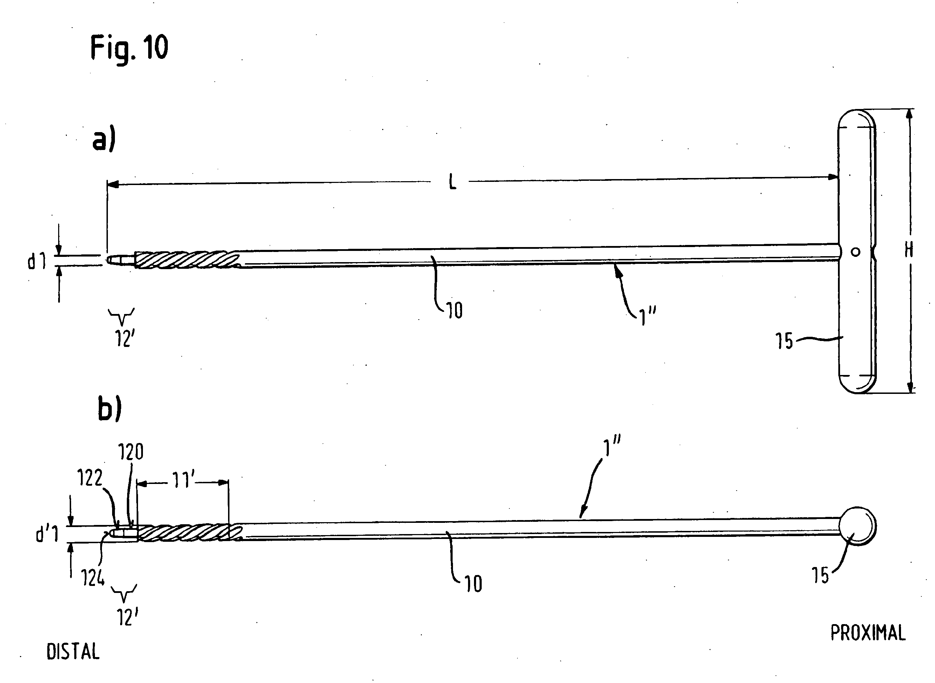

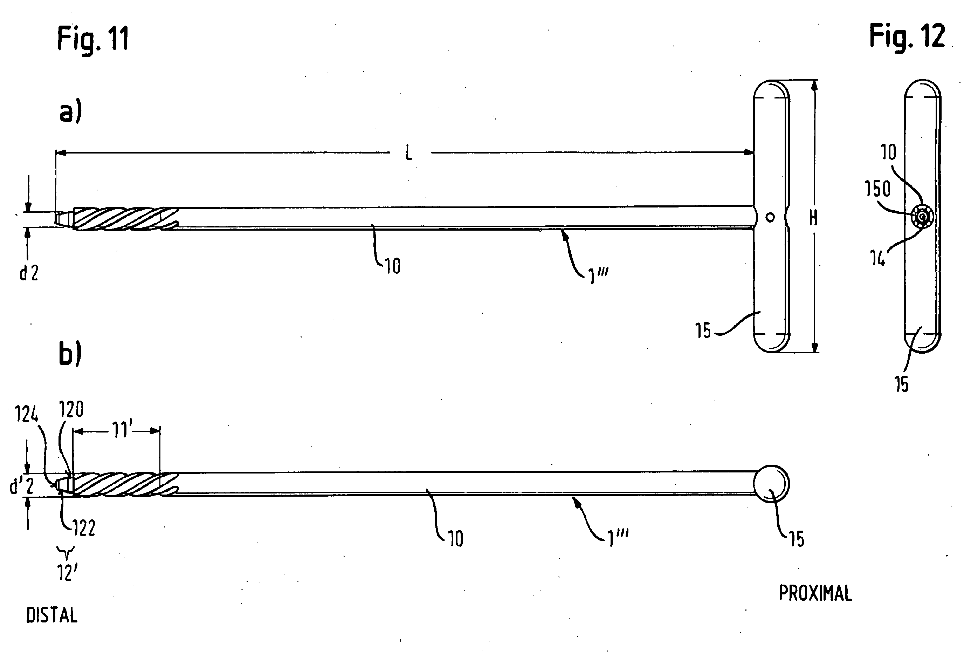

Figures 10 and 11 show a set of drills in another embodiment of the present disclosure. In particular, Figures 10a and 10b show a first drill of the set of drills and Figures 11a and 11b show a second surgical drill of the set of drills.

The surgical drill 1'' shown in Figures 10 a) and 10 b) exhibits a first outer diameter d'1 of the cutting section 11' of 4mm. The diameter d1 of the non-cutting protection tip 12' is 2mm in this embodiment. The length of the cutting section 11' in the axial direction is 25mm.

According to the embodiment shown in Figures 10a and 10b, the non-cutting protection tip 12' comprises a cylindrical section 120 that is situated distally of the cutting section 11'. Distally of the cylindrical section 120, a tapered section 122 is provided which is followed in the distal direction by a rounded section 124. The tapered section 122 in the embodiment shown has a basically conical shape. The rounded section 124 has a substantially hemispherical shape but could also have an elliptical, parabolic or any other shape serving the purpose of further reducing the impact of the non-cutting protection tip on any sort of soft tissue or nerves.

As mentioned above, the rounded section 124 further improves the protection characteristics of the non-cutting protection tip 12' in that the smoothly rounded edges do not cut or damage nerves, tissue and/or dura. The tapered section 122 is used for easily and securely inserting the surgical drill 1'' into an already drilled hole and/or an already drilled canal in the bone. The cylindrical section 120 is used to centre the surgical drill 1'' and in particular the drill member 10 securely about such a readily drilled hole.

The length of the drill member 10 between the distal end of the non-cutting protection tip 12' and the handle 15, namely the length L, is about 200 mm. The handle 15 has a lateral overall extension of about 75 mm.

Figures 11 a) and 11 b) show basically the same structure of a surgical drill 1''' as that of the surgical drill 1'' shown in Figures 10 a) and 10 b). In particular, the drill member 10 also comprises a cutting section 11' and a non-cutting protection tip 12' whereas the non-cutting protection tip 12' includes, from proximal to distal, a cylindrical section 120, a tapered section 122 and a rounded section 124. The length of the drill member 10, namely the length L, is approximately 200 mm and the lateral overall extension H of the handle 15 is approximately 75 mm. As in the case of the surgical drill 1'' shown in Figures 10a and 10b, the handle 15 is a removable handle that can be removed from the drill member 10.

The dimensions in the axial direction of the cutting section 11' are, as in the case of the surgical drill 1'' shown in Figures 10 a) and 10 b), 25 mm.

However, the diameter of the surgical drill 1''' in the embodiment shown in Figures 11 a) and 11 b) are different from the diameters of the surgical drill shown in Figures 10 a) and 10 b). In particular, in the embodiment of the surgical drill 1''', the outer diameter d'2 of the cutting section 11' is 6 mm and the outer diameter d2 of the non-cutting protection tip 12' is 4 mm.

Thus, the non-cutting protection tip 12' of the surgical drill 1''' according to Figures 11 a) and 11 b) can be inserted into a hole or canal that was previously drilled with the surgical drill 1'' according to Figures 10 a) and 10 b). The surgical drill 1''' according to Figures 11 a) and 11 b) is then correctly centred about the hole and/or the channel previously drilled by the surgical drill 1''.

Figure 12 shows a top view on the handle 15 of the surgical drills 1'' and 1''' of Figures 10 a), 10 b), 11 a) and 11 b). In particular, a locking mechanism 150 can be appreciated that enables the surgeon to lock the removable handle 15 to the drill member 10.

Furthermore, in the centre of the drill member 10, the guide channel 14 for the insertion of a guide wire is shown. The surgical drill 1'' of Figures 10 a) and 10 b) as well as the surgical drill 1''' of Figures 11 a) and 11 b) both comprise a guide channel 14 that extends from the distal end to the proximal end and that is used to insert a guide wire through the drill member 10 from the distal to the proximal end. The inner diameter of the guide channel 14 is basically the same for all surgical drills of the embodiment shown.

Accordingly, the embodiments shown in Figures 10 a), 10 b), 11 a) and 11 b) show an exemplary set of surgical drills comprising of at least two surgical drills 1'', 1''' of different outer diameters d'1, d'2 of the respective cutting sections 11', as well as different outer diameters d1; d2 of the respective non-cutting protection tips 12'.

## Claims

1. A surgical drill (1, 1') for removing bone (65, 67) to access the spinal canal (62), the surgical drill including a drill member (10) comprising a cutting section (11, 11', 11'') for cutting bone, said cutting section being situated at a distal end of the drill member, wherein the drill member comprises a non-cutting protection tip (12) for protecting nerves, tissue and/or dura from being cut, the non-cutting protection tip being situated distally of the cutting section, **characterized in that** the drill member comprises a guide channel (13) extending in the axial direction of the drill member such that a guide wire (4') can be inserted into the guide channel through the drill member and **in that** the cutting section has a cutting end face adjacent to the non-cutting protection tip such that the longitudinal axis of the drill member is oriented perpendicular to the cutting end face.

2. The surgical drill according to claim 1, **characterized in that** the cutting section has a first outer diameter (d') and the non-cutting protection tip has a second outer diameter (d) which is smaller than the first outer diameter of the cutting section.

3. The surgical drill according to claim 2, **characterized in that** the non-cutting protection tip has a second outer diameter (d) which is 1 mm to 2 mm smaller than the first outer diameter of the cutting section.

4. The surgical drill according to any one of the preceding claims, **characterized in that** the non-cutting protection tip comprises at least one substantially cylindrical section (120) and at least one substantially tapered section (122) situated distally of the substantially cylindrical section.

5. The surgical drill according to claim 4, **characterized in that** the non-cutting protection tip comprises at least one substantially rounded section (124) situated distally of the substantially tapered section.

6. The surgical drill according to any one of the preceding claims, **characterized in that** the non-cutting protection tip is blunt.

7. The surgical drill according to claim 6, **characterized in that** the non-cutting protection tip has at least one polished and/or smooth surface section, in particular a mechanically polished surface section and/or an electro-polished surface section.

8. The surgical drill according to any one of the preceding claims, **characterized in that** the non-cutting protection tip carries a smooth coating, in particular a lubricious, a low friction and/or a PTFE coating.

9. The surgical drill according to any one of the preceding claims, **characterized in that** the non-cutting protection tip has a length (1) in the distal direction of at least 5 mm, preferably between 5 mm and 15 mm, preferred of 5mm, 10mm or 15mm.

10. The surgical drill according to any one of the preceding claims, **characterized in that** it comprises a sleeve (2) having a sleeve channel (20) for receiving the drill member (10) such that said drill member can rotate when inserted into the sleeve.

11. The surgical drill according to claim 10, **characterized in that** the sleeve has an inner threading (222) on its proximal end and the drill member has an outer threading (122) on its proximal end such that the drill member can be advanced relative to the sleeve by relative rotation of the drill member to the sleeve.

12. The surgical drill according to any one of the preceding claims, **characterized in that** the guide channel extends from the distal end of the drill member to the proximal end of the drill member.

13. The surgical drill according to any one of the preceding claims, **characterized in that** the cutting section is at least one of: a burr, a trephine, a milling cutter, a reamer or a trepan (11, 11").

14. The surgical drill according to any one of the preceding claims, **characterized in that** the cutting section comprises a drill threading (11').

15. The surgical drill according to any one of the preceding claims, **characterized in that** a handle (15), a removable handle, and/or a connector to a power drill is situated at a proximal end of the drill member.

16. A set of surgical drills comprising at least a first and a second surgical drill (1) according to any one of the preceding claims, wherein the outer diameter of the cutting section (11) of the first surgical drill is equal to or larger than the outer diameter of the non-cutting protection tip (12) of the second surgical drill.

17. The set of surgical drills according to claim 16, **characterized in that** the diameter of the non-cutting protection tip of the second drill has a diameter such that it can be inserted into a hole drilled by the cutting section of the first drill.

18. The set of surgical drills according to claim 16 or 17, **characterized in that** a plurality of surgical drills is provided, the drills having diameters (d') of the cutting section of 3mm, 4mm, 5mm, 6mm, 7mm, 8mm, 9mm, 10mm, 11mm and 12mm, or 3,5mm, 4,5mm, 5,5mm, 6 , 5mm, 7 , 5mm, 8,5mm, 9,5mm, 10,5mm, 11, 5mm and 12,5mm, respectively.

19. The set of surgical drills according to claim 16 or 17, **characterized in that** a plurality of surgical drills is provided, the drills having diameters (d') of the cutting sections of 4mm, 6mm, 8mm and 10mm, and the diameters of the respective non-cutting protection tips being 2mm, 4mm, 6mm and 8mm, respectively.

20. The set of surgical drills according to any one of claims 16 to 19, **characterized in that** the lengths (1) of the non-cutting protection tips of all surgical drills are the equal.

21. The set of surgical drills according to any one of claims 16 to 20, **characterized in that** the surgical drills comprise sleeves such that the sleeve of each subsequent drill can be put over the sleeve of the previous drill, respectively.

22. A system for cutting bone, the system comprising at least one surgical drill according to any one of claims 1 to 15 and a working cannula (9) for insertion into a body.

23. The system according to claim 22, **characterized in that** it comprises a needle (3) comprising a trocar, in particular an 18-gauge needle, for positioning a pilot notch in a bone of a body and a guide wire (4) to be inserted through a cavity of the needle.

24. The system according to claims 22 or 23, **characterized in that** it comprises a dilating rod (5) for the dilation of a channel in body tissue.

25. The system according to any one of the claims 22 to 24, **characterized in that** it comprises a Yamshidi needle with a Yamshidi sleeve (7) and a Yamshidi trocar for introducing a pilot hole in a bone and a drill (8) for insertion into the Yamshidi sleeve.

26. The system according to any one of the claims 22 to 25, **characterized in that** it comprises a set of surgical drills according to any one of claims 16 to 21.

## Patentansprüche

1. Ein chirurgischer Bohrer (1, 1') zum Entfernen von Knochen (65, 67) und um Zugang zu dem Spinalkanal (62) zu haben, der chirurgische Bohrer einschließend ein Bohrteil (10), umfassend einen Schneidabschnitt (11, 11', 11'') zum Schneiden von Knochen, der Schneidabschnitt gelegen an einem distalen Ende des Bohrteils, wobei das Bohrteil eine nicht schneidende Schutzspitze (12) umfasst, zum Schützen von Nerven, Gewebe und/oder Dura vor dem geschnitten zu werden, die nicht schneidende Schutzspitze distal vom Schneidabschnitt gelegen, **dadurch gekennzeichnet, dass** das Bohrteil einen Führungskanal (13) umfasst, sich in der axialen Richtung des Bohrteils erstreckend, so dass ein Führungsdraht (4') in den Führungskanal durch das Bohrteil eingeführt werden kann und **dadurch**, dass der Schneidabschnitt eine Schneidendfläche aufweist, angrenzend an die nicht schneidende Schutzspitze, so dass die Längsachse des Bohrteils senkrecht zu der Schneidendfläche ausgerichtet ist.

2. Der chirurgische Bohrer gemäß Anspruch 1, **dadurch gekennzeichnet, dass** der Schneidabschnitt einen ersten äußeren Durchmesser (d') aufweist und die nicht schneidende Schutzspitze einen zweiten äußeren Durchmesser (d) aufweist, der kleiner ist als der erste äußere Durchmesser des Schneidabschnitts.

3. Der chirurgische Bohrer gemäß Anspruch 2, **dadurch gekennzeichnet, dass** die nicht schneidende Schutzspitze einen zweiten äußeren Durchmesser (d) aufweist, der 1 mm bis 2 mm kleiner ist als der erste äußere Durchmesser des Schneidabschnitts.

4. Der chirurgische Bohrer gemäß einem der vorstehenden Ansprüche, **dadurch gekennzeichnet, dass** die nicht schneidende Schutzspitze mindestens einen im Wesentlichen zylindrischen Abschnitt (120) umfasst und mindestens einen im Wesentlichen konischen Abschnitt (122), distal von dem im Wesentlichen zylindrischen Abschnitt gelegen.

5. Der chirurgische Bohrer gemäß Anspruch 4, **dadurch gekennzeichnet, dass** die nicht schneidende Schutzspitze mindestens einen im Wesentlichen abgerundeten Abschnitt (124) umfasst, distal von dem im Wesentlichen konischen Abschnitt gelegen.

6. Der chirurgische Bohrer gemäß einem der vorstehenden Ansprüche, **dadurch gekennzeichnet, dass** die nicht schneidende Schutzspitze stumpf ist.

7. Der chirurgische Bohrer gemäß Anspruch 6, **dadurch gekennzeichnet, dass** die nicht schneidende Schutzspitze mindestens einen polierten und/oder glatten Oberflächenabschnitt aufweist, insbesondere einen mechanisch polierten Oberflächenabschnitt und/oder einen elektropolierten Oberflächenabschnitt.

8. Der chirurgische Bohrer gemäß einem der vorstehenden Ansprüche, **dadurch gekennzeichnet, dass** die nicht schneidende Schutzspitze eine glatte Beschichtung trägt, insbesondere eine schlüpfrige, eine friktionsarme und/oder eine PTFE-Beschichtung.

9. Der chirurgische Bohrer gemäß einem der vorstehenden Ansprüche, **dadurch gekennzeichnet, dass** die nicht schneidende Schutzspitze eine Länge (1) in der distalen Richtung aufweist, von mindestens 5 mm, vorzugsweise zwischen 5 mm und 15 mm, bevorzugt 5 mm, 10 mm oder 15 mm.

10. Der chirurgische Bohrer gemäß einem der vorstehenden Ansprüche, **dadurch gekennzeichnet, dass** er eine Hülse (2) umfasst, aufweisend einen Hülsenkanal (20) zum Aufnehmen des Bohrteils (10), so dass sich das Bohrteil drehen kann, wenn in die Hülse eingeführt.

11. Der chirurgische Bohrer gemäß Anspruch 10, **dadurch gekennzeichnet, dass** die Hülse ein inneres Gewinde (222) an ihrem proximalen Ende aufweist und das Bohrteil ein äußeres Gewinde (122) an seinem proximalen Ende aufweist, so dass das Bohrteil relativ zur Hülse vorgeschoben werden kann, durch Drehen des Bohrteils relativ zur Hülse.

12. Der chirurgische Bohrer gemäß einem der vorstehenden Ansprüche, **dadurch gekennzeichnet, dass** sich der Führungskanal von dem distalen Ende des Bohrteils zu dem proximalen Ende des Bohrteils erstreckt.

13. Der chirurgische Bohrer gemäß einem der vorstehenden Ansprüche, **dadurch gekennzeichnet, dass** der Schneidabschnitt mindestens eines ist von: einem Schnittgrad, einem Trokar, einem Fräser, einer Reibahle oder einem Trepan (11, 11").

14. Der chirurgische Bohrer gemäß einem der vorstehenden Ansprüche, **dadurch gekennzeichnet, dass** der Schneidabschnitt eine Gewindebohrung (11') umfasst.

15. Der chirurgische Bohrer gemäß einem der vorstehenden Ansprüche, **dadurch gekennzeichnet, dass** ein Griffstück (15), ein entfernbares Griffstück und/oder ein Verbinder zu einer Bohrmaschine an einem proximalen Ende des Bohrteils gelegen ist.

16. Ein Set von chirurgischen Bohrern, umfassend mindestens einen ersten und einen zweiten chirurgischen Bohrer (1) gemäß einem der vorstehenden Ansprüche, wobei der äußere Durchmesser des Schneidabschnitts (11) des ersten chirurgischen Bohrers gleich oder größer ist als der äußere Durchmesser der nicht schneidenden Schutzspitze (12) des zweiten chirurgischen Bohrers.

17. Das Set von chirurgischen Bohrern gemäß Anspruch 16, **dadurch gekennzeichnet, dass** der Durchmesser der nicht schneidenden Schutzspitze des zweiten Bohrers einen Durchmesser aufweist, so dass sie in ein Loch eingeführt werden kann, gebohrt durch den Schneidabschnitt des ersten Bohrers.

18. Das Set von chirurgischen Bohrern gemäß Anspruch 16 oder 17, **dadurch gekennzeichnet, dass** eine Vielzahl von chirurgischen Bohrern bereitgestellt ist, die Bohrer Durchmesser (d') des jeweiligen Schneidabschnitts von 3 mm, 4 mm, 5 mm, 6 mm, 7 mm, 8 mm, 9 mm, 10 mm, 11 mm und 12 mm, oder 3,5 mm, 4,5 mm, 5,5 mm, 6,5 mm, 7,5 mm, 8,5 mm, 9,5 mm, 10,5 mm, 11,5 mm und 12,5 mm aufweisend.

19. Das Set von chirurgischen Bohrern gemäß Anspruch 16 oder 17, **dadurch gekennzeichnet, dass** eine Vielzahl von chirurgischen Bohrern bereitgestellt ist, die Bohrer Durchmesser (d') des Schneidabschnitts von 4 mm, 6 mm, 8 mm und 10 mm aufweisend und die Durchmesser der nicht schneidenden Schutzspitzen sind jeweils 2 mm, 4 mm, 6 mm und 8 mm.

20. Das Set von chirurgischen Bohrern gemäß einem der Ansprüche 16 bis 19, **dadurch gekennzeichnet, dass** die Längen (1) der nicht schneidenden Schutzspitzen von allen chirurgischen Bohrern die gleichen sind.

21. Das Set von chirurgischen Bohrern gemäß einem der Ansprüche 16 bis 20, **dadurch gekennzeichnet, dass** die chirurgischen Bohrer Hülsen umfassen, so dass die Hülse von jedem nachfolgenden Bohrer über die Hülse des jeweils vorhergehenden Bohrers gesteckt werden kann.

22. Ein System zum Schneiden von Knochen, das System umfassend mindestens einen chirurgischen Bohrer gemäß einem der Ansprüche 1 bis 15 und eine Arbeitskanüle (9) zum Einführen in einen Körper.

23. Das System gemäß Anspruch 22, **dadurch gekennzeichnet, dass** es eine Nadel (3) umfasst, umfassend einen Trokar, insbesondere eine 18-Gauge-Nadel, zum Positionieren einer Steuerkerbe in einem Knochen eines Körpers und einen Führungsdraht (4), um durch einen Hohlraum der Nadel eingeführt zu werden.

24. Das System gemäß Anspruch 22 oder 23, **dadurch gekennzeichnet, dass** es einen Dilatierstab (5) zur Dilatation eines Kanals im Körpergewebe umfasst.

25. Das System gemäß einem der Ansprüche 22 bis 24, **dadurch gekennzeichnet, dass** es eine Yamshidi-Nadel umfasst mit einer Yamshidi-Hülse (7) und einem Yamshidi-Trokar zum Einbringen eines Führungslochs in einen Knochen und einen Bohrer (8) zum Einschieben in die Yamshidi-Hülse.

26. Das System gemäß einem der Ansprüche 22 bis 25, **dadurch gekennzeichnet, dass** es ein Set von chirurgischen Bohrern gemäß einem der Ansprüche 16 bis 21 umfasst.

## Revendications

1. Foret chirurgical (1, 1') destiné à retirer de l'os (65, 67) pour accéder au canal rachidien (62), le foret chirurgical comportant un élément de forage (10) comprenant une section de coupe (11, 11', 11'') pour couper de l'os, ladite section de coupe est placée au niveau d'une extrémité distale de l'élément de forage, où l'élément de forage comprend une pointe (12) de protection non coupante pour éviter que des nerfs, des tissus et/ou d'un sac dural ne soient coupés, la pointe de protection non coupante est placée de manière distale à la section de coupe, **caractérisé en ce que** l'élément de forage comprend un canal de guidage (13) s'étendant dans la direction axiale de l'élément de forage de façon à ce qu'un fil-guide (4') puisse être inséré dans le canal de guidage à travers l'élément de forage et **en ce que** la section de coupe a une face d'extrémité de coupe adjacente à la pointe de protection non coupante de façon à ce que l'axe longitudinal de l'élément de forage soit orienté perpendiculairement à la face d'extrémité de coupe.

2. Foret chirurgical selon la revendication 1, **caractérisé en ce que** la section de coupe a un premier diamètre externe (d') et la pointe de protection non coupante a un deuxième diamètre externe (d) qui est plus petit que le premier diamètre externe de la section de coupe.

3. Foret chirurgical selon la revendication 2, **caractérisé en ce que** la pointe de protection non coupante a un deuxième diamètre externe (d) qui est de 1 mm à 2 mm plus petit que le premier diamètre externe de la section de coupe.

4. Foret chirurgical selon l'une quelconque des revendications précédentes, **caractérisé en ce que** la pointe de protection non coupante comprend au moins une section essentiellement cylindrique (120) et au moins une section essentiellement conique (122) placée de manière distale à la section essentiellement cylindrique.

5. Foret chirurgical selon la revendication 4, **caractérisé en ce que** la pointe de protection non coupante comprend au moins une section essentiellement arrondie (124) placée de manière distale de la section essentiellement conique.

6. Foret chirurgical selon l'une quelconque des revendications précédentes, **caractérisé en ce que** la pointe de protection non coupante est émoussée.

7. Foret chirurgical selon la revendication 6, **caractérisé en ce que** la pointe de protection non coupante a au moins une section de surface lisse et/ou polie, en particulier une section de surface mécaniquement polie et/ou une section de surface électropolie.

8. Foret chirurgical selon l'une quelconque des revendications précédentes, **caractérisé en ce que** la pointe de protection non coupante porte un revêtement lisse, en particulier un revêtement lubrifié, un revêtement à faible coefficient de frottement et/ou un revêtement en PTFE.

9. Foret chirurgical selon l'une quelconque des revendications précédentes, **caractérisé en ce que** la pointe de protection non coupante a une longueur (1) dans la direction distale d'au moins 5 mm, de préférence entre 5 mm et 15 mm, de préférence 5 mm, 10 mm ou 15 mm.

10. Foret chirurgical selon l'une quelconque des revendications précédentes, **caractérisé en ce qu'**il comprend un manchon (2) ayant un canal (20) de manchon pour recevoir l'élément de forage (10) de façon à ce que ledit élément de forage puisse tourner lorsqu'il est inséré dans le manchon.

11. Foret chirurgical selon la revendication 10, **caractérisé en ce que** le manchon a un taraudage intérieur (222) sur son extrémité proximale et l'élément de forage a un filetage extérieur (122) sur son extrémité proximale de façon à ce que l'élément de forage puisse avancer par rapport au manchon par la rotation relative de l'élément de forage par rapport au manchon.

12. Foret chirurgical selon l'une quelconque des revendications précédentes, **caractérisé en ce que** le canal de guidage s'étend de l'extrémité distale de l'élément de forage à l'extrémité proximale de l'élément de forage.

13. Foret chirurgical selon l'une quelconque des revendications précédentes, **caractérisé en ce que** la section de coupe est au moins l'une : d'une fraise, d'un trépan, d'une fraise à rainer, d'un alésoir ou d'un trépan (11, 11'').

14. Foret chirurgical selon l'une quelconque des revendications précédentes, **caractérisé en ce que** la section de coupe comprend un filetage de forage (11').

15. Foret chirurgical selon l'une quelconque des revendications précédentes, **caractérisé en ce qu'**une poignée (15), une poignée amovible, et/ou un connecteur à un foret électrique sont placés au niveau d'une extrémité proximale de l'élément de forage.

16. Ensemble de forets chirurgicaux comprenant au moins des premier et deuxième forets chirurgicaux (1) selon l'une quelconque des revendications précédentes, dans lequel le diamètre extérieur de la section de coupe (11) du premier foret chirurgical est supérieur ou égal au diamètre extérieur de la pointe (12) de protection non coupante du deuxième foret chirurgical.

17. Ensemble de forets chirurgicaux selon la revendication 16, **caractérisé en ce que** le diamètre de la pointe de protection non coupante du deuxième foret a un diamètre tel qu'il peut être inséré dans un trou foré par la section de coupe du premier foret.

18. Ensemble de forets chirurgicaux selon la revendication 16 ou 17, **caractérisé en ce qu'**une pluralité de forets chirurgicaux sont prévus, les forets ayant des diamètres (d') de la section de coupe de 3 mm, 4 mm, 5 mm, 6 mm, 8 mm, 9 mm, 10 mm, 11 mm et 12 mm, ou de 3,5 mm, 4,5 mm, 5,5 mm, 6,5 mm, 7,5 mm, 8, 5 mm, 9,5 mm, 10,5 mm, 11,5 mm et 12,5 mm, respectivement.

19. Ensemble de forets chirurgicaux selon la revendication 16 ou 17, **caractérisé en ce qu'**une pluralité de forets chirurgicaux sont prévus, les forets ayant des diamètres (d') des sections de coupe de 4 mm, 6 mm, 8 mm et 10 mm, et les diamètres des pointes de protection non coupantes sont de 2 mm, 4 mm, 6 mm et 8 mm, respectivement.

20. Ensemble de forets chirurgicaux selon l'une quelconque des revendications 16 à 19, **caractérisé en ce que** les longueurs (1) des pointes de protection non coupantes de tous les forets chirurgicaux sont égales.

21. Ensemble de forets chirurgicaux selon l'une quelconque des revendications 16 à 20, **caractérisé en ce** les forets chirurgicaux comprennent des manchons de façon à ce que le manchon de chaque foret qui suit puisse être placé sur le manchon du foret précédent, respectivement.

22. Système pour couper un os, le système comprenant au moins un foret chirurgical selon l'une quelconque des revendications 1 à 15 et une canule de travail (9) pour l'insérer dans un corps.

23. Système selon la revendication 22, **caractérisé en ce qu'**il comprend une aiguille (3) comprenant un trocart, en particulier une aiguille 18 gauge, pour positionner une encoche pilote dans un os d'un corps et un fil-guide (4) à insérer à travers une cavité de l'aiguille.

24. Système selon les revendications 22 ou 23, **caractérisé en ce qu'**il comprend une tige de dilatation (5) pour dilater un canal dans un tissu corporel.

25. Système selon l'une quelconque des revendications 22 à 24, **caractérisé en ce qu'**il comprend une aiguille de Yamshidi avec un manchon (7) de Yamshidi et un trocart de Yamshidi pour introduire un trou pilote dans un os et un foret (8) pour l'insérer dans le manchon de Yamshidi.

26. Système selon l'une quelconque des revendications 22 à 25, **caractérisé en ce qu'**il comprend un ensemble de forets chirurgicaux selon l'une quelconque des revendications 16 à 21.
